(19) Europäisches Patentamt
European Patent Office
Office européen des brevets

(11) EP 1 624 756 B1

(12) EUROPÄISCHE PATENTSCHRIFT

(45) Veröffentlichungstag und Bekanntmachung des Hinweises auf die Patenterteilung:
**24.06.2009 Patentblatt 2009/26**

(21) Anmeldenummer: **04729371.7**

(22) Anmeldetag: **24.04.2004**

(51) Int Cl.:
***A01N 53/00*** *(2006.01)*

(86) Internationale Anmeldenummer:
**PCT/EP2004/004359**

(87) Internationale Veröffentlichungsnummer:
**WO 2004/098290 (18.11.2004 Gazette 2004/47)**

(54) **MITTEL ZUM BEKÄMPFEN VON PARASITEN AN TIEREN**

AGENTS FOR CONTROLLING PARASITES ON ANIMALS

AGENTS POUR LUTTER CONTRE DES PARASITES DES ANIMAUX

(84) Benannte Vertragsstaaten:
**AT BE BG CH CY CZ DE DK EE ES FI FR GB GR HU IE IT LI LU MC NL PL PT RO SE SI SK TR**
Benannte Erstreckungsstaaten:
**HR**

(30) Priorität: **08.05.2003 DE 10320505**

(43) Veröffentlichungstag der Anmeldung:
**15.02.2006 Patentblatt 2006/07**

(73) Patentinhaber: **Bayer Animal Health GmbH**
**51368 Leverkusen (DE)**

(72) Erfinder:
- **SIRINYAN, Kirkor**
**51467 Bergisch Gladbach (DE)**
- **TURBERG, Andreas**
**42781 Haan (DE)**

(56) Entgegenhaltungen:
**EP-A- 0 596 317**
**WO-A-86/03374**
**WO-A-02/087338**
**AU-B- 666 399**

- **DATABASE BIOSIS [Online] BIOSCIENCES INFORMATION SERVICE, PHILADELPHIA, PA, US; 1995, ONO YOSHIHIRO ET AL: "Synergistic effect of synthetic synergists on pyrethroids against adults of the cat flea, Ctenocephalides felis (Bouche)" XP002299303 Database accession no. PREV199698561306 in der Anmeldung erwähnt & JAPANESE JOURNAL OF SANITARY ZOOLOGY, Bd. 46, Nr. 1, 1995, Seiten 25-30, ISSN: 0424-7086 in der Anmeldung erwähnt**
- **DATABASE WPI Section Ch, Week 199539 Derwent Publications Ltd., London, GB; Class C03, AN 1995-299442 XP002299306 & JP 07 196420 A (SUMITOMO CHEM CO LTD) 1. August 1995 (1995-08-01) -& DATABASE CHEMABS [Online] CHEMICAL ABSTRACTS SERVICE, COLUMBUS, OHIO, US; XP002299304 gefunden im STN-INTERNATIONAL Database accession no. 123:249228**
- **R.H.ROBERTS ET AL.: "Effects of Additives on the Toxicity of Pyrethrins to Stable Flies and Horn Flies" JOURNAL OF ECONOMIC ENTOMOLOGY., Bd. 56, Nr. 5, Oktober 1963 (1963-10), Seiten 699-702, XP002299302 USENTOMOLOGICAL SOCIETY OF AMERICA. COLLEGE PARK, MARYLAND.**
- **DATABASE CHEMABS [Online] CHEMICAL ABSTRACTS SERVICE, COLUMBUS, OHIO, US; XP002299305 gefunden im STN-INTERNATIONAL Database accession no. 109: 124423 & JP 63 126805 A (NIHON TOKUSHU NOYAKU SEIZO) 30. Mai 1988 (1988-05-30)**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung, betrifft neue Mittel zur Bekämpfung von Parasiten an Tieren, enthaltend Flumethrin sowie MGK 264 in einem Gewichtsverhältnis der Komponenten von mindestens 1:20 und höchstens 1:100; die Mittel enthalten gegebenenfalls einen weiteren insektiziden und/oder acariziden Wirkstoff.

Zur Anwendung zum Teil schwer wasserlöslicher Wirkstoffe in Form von dermal applizierbaren Flüssigformulierungen ist es notwendig, homogene Lösungen oder Emulsionen auf Basis von organischen Lösungsmitteln und insektiziden Wirkstoffen herzustellen. Dazu werden die Wirkstoffe zumeist in organischen Lösungsmitteln wie Isopropanol, 2-Butoxy-ethylacetat, Ethylenglykoldiacetat gelöst und gegebenenfalls mit weiteren Zusatzstoffen vermischt. Die Herstellung solcher Formulierungen ist in US 4 874 753, EP-A 137 627 und GB 2 135 886 beschrieben. Die Nachteile der besagten Systeme liegen z.B. bei Verwendung von Wirkstoffen aus der Klasse der Pyrethrine sowie Pyrethroide, insbesondere $\alpha$-Cyanopyrethroide darin, dass sie zu schweren Hautirritationen führen und ferner eine geringe Langzeitwirkung aufweisen. Es ist wünschenswert, diese Formulierungen durch solche zu ersetzen, die hautverträglich sowie toxikologisch unbedenklich sind und sich durch eine Langzeitwirkung von mehreren Wochen auszeichnen.

Um den besagten Nachteil beispielsweise der bekannten Pyrethroide und Pyrethrine zu beheben, wird in AU-627 847 und EP-A 413 610 vorgeschlagen, diese Wirkstoffe in hochsiedenden Lösungsmitteln wie Monopropylenglykol zu lösen, die zusätzlich noch natürliche, hautverträgliche Öle wie Pinienöl, Sonnenblumenöl oder Sojaöl enthalten. Der WO 91/13545 kann entnommen werden, dass gut wirksame, hautverträgliche Flüssigformulierungen hergestellt werden können, indem man die besagten Wirkstoffe in Mengen von >50% in aliphatischen Lösungsmitteln wie 2-(2-Butoxyethoxy) ethanol oder 2-(2-Methoxy-ethoxy)ethanol löst. Der Nachteil dieser Formulierungen liegt darin, dass sie den Einsatz größerer Wirkstoff-Mengen benötigen und zudem bei sensiblen Tierrassen zu Hautirritationen fuhren. Um durch Einsatz geringer Wirkstoffinengen eine akzeptable biologische Wirkung zu erreichen, wird in der Patentschrift US 5 466 458 die Verwendung von Emulsionen auf Basis der besagten Wirkstoffe mit den langkettigen, aliphatischen Aminen oder Alkoholen wie Hexadecan-1-ol, 1-Octadecylamine vorgeschlagen. Die Verwendung der langkettigen Amine hat den Nachteil, dass sie die besagten Wirkstoffe im Laufe der Zeit abbauen. Die Formulierungen auf Basis langkettiger Alkohole weisen in den meisten Fällen keine ausreichende Langzeitwirkung auf.

Ferner wird in der WO 01/35739 vorgeschlagen, die bezüglich Hautirritation kritischen Pyrethroide, insbesondere $\alpha$-Cyanopyrethroide, mit Polysiloxanen, die zusätzlich quarternäre Ammoniumgruppen enthalten, zu kombinieren. Diese elegante Zubereitungsform hat jedoch den Nachteil, dass sie den Einsatz größerer Pyrethroid-Mengen erfordert. Diese Tatsache kann in vielen Fällen zur Zieltier- oder Umwelt-Unverträglichkeit fuhren.

Der Literatur kann entnommen werden, dass man synthetische oder natürliche Pyrethroide mit organischen Synergisten wie Piperonylbutoxid (PBO), (2-(2-Ethylhexyl)-3a,4,7,7a-tetrahydro-4,7-methano-1H-isoindol-1,3(2H)-dion (MGK 264), S,S,S-Tributylphosphorotrithioat (DEF) oder Synepirin kombinieren kann [s. bespielsweise JOURNAL OF ECONOMIC ENTOMOLOGY, (1994 Aug) 87 (4) 879-84, 1994; JOURNAL OF ECONOMIC ENTOMOLOGY, (1987 Aug) 80 (4) 728-32 oder India Chemosphere, (Nov., 1997) Vol. 35, No. 10, pp. 2365-2374. ISSN: 0045-6535, Japanese Journal of Sanitary Zoology, (1995) Vol. 46, No. 1, pp. 25-30. ISSN: 0424-7086. 1995 sowie J ECON ENTOMOL, (1987) 80 (6), 1117-1121. CODEN: JEENAI. ISSN: 0022-0493. 1987)]. Ferner kann der o.a. Literatur entnommen werden, dass die Wirksamkeit der Pyrethroidhaltigen Zubereitungen gegen adulte Flöhe verbessert werden kann, wenn man Pyrethroide mit den besagten Synergisten in Mengen 1:5 bis zu max. 1:20 kombiniert. Der Literatur [siehe beispielsweise DEP. ENTOMOL., UNIV. GEORGIA, COASTAL PLAIN EXP. STN., TEFLON, GA. 31793 oder India Chemosphere, (1998) 36/15 (3055-3060) 1998] kann entnommen werden, dass eine maximale Wirksamkeitserhöhung bei einem Mengenverhältnis Wirkstoff zu Synergist von 1:5 erreicht wird (z.B. bei Permethrin/MGK-264 oder Fenvalerate/PBO).

Es ist weiterhin bekannt, dass Shampoos enthaltend Dipropylpyridin-2,5-dicarboxylat, MGK 264, Piperonylbutoxid, und Pyrethrine zur Bekämpfung von Flöhen bei Kleintieren verwendet werden können [siehe beispielsweise Wang I.-H.; Moorman R.; Burleson J.I-H. Wang, Journal of Liquid Chromatography and Related Technologies, (1996) 19/20 (3293-3304)].

Des weiteren ist bekannt, dass Carbamate wie Propoxur in Kombination mit PPO und MGK 264 in Mengenverhältnissen 1,00 : 0,04: 0,1 sich zur Umgebungsbehandlung eignen (s. beispielsweise Firmenprospekt der Fa. Sano Bruns Enterprises Ltd. Israel, 1990 AO1N-047/44).

AU-B-47404/93 beschreibt Mischungen zum Bekämpfung von Ektoparasiten, die Pyrethroide wie z.B. Flumethrin und Synergisten wie z.B. MGK 264 in Verhältnissen von 1:2 bis 1:10 enthalten.

In 1999 US 0 124 306 werden Kombinationen mit Imidacloprid und/oder Fipronil und/oder Pyrethroiden zur Schädlingsbekämpfung im Agrobereich, beschrieben. Ferner werden in EP-A-981 956 (US-6 080 796) Schäume auf Basis der o.a. Wirkstoffe sowie in der Patentanmeldung EP-A-981 955 (US-6033 731) Polymerlegierungen, welche aus Suspensionen oder Emulsionen der Wirkstoffe Imidacloprid und Permethrin hergestellt werden, zur Parasitenbekämpfung beschrieben.

Alle genannten Zubereitungsformen haben den Nachteil, dass sie, bei einer akzeptablen Applikationsform, zur Bekämpfung von Ektoparasiten wie Flöhen, Zecken und Mücken für eine Dauer von mindestens drei, vorzugsweise

jedoch von vier Wochen nicht geeignet sind und zudem den Einsatz von größeren Wirkstoff Mengen erfordern.

Der vorliegenden Erfindung liegt die Aufgabe zur Grunde, Mittel enthaltend Flumethrin bereitzustellen, die sich zur Bekämpfung von Parasiten, vorzugsweise Ektoparasiten, an Tieren eignen. Solche Zubereitungen sollten sich durch hohe parasitizide Wirksamkeit und gute Verträglichkeit beim behandelten Tier auszeichnen. Darüberhinaus sind auch gute Anwender- und Umweltverträglichkeit von Bedeutung. Es sollten sich flüssige Zubereitungen realisieren lassen, welche die elegante Spot on Applikation ermöglichen.

Überraschenderweise wird diese Aufgabe dadurch gelöst, dass man Flumethrin in Kombination mit dem Synergisten MGK 264 entgegen der bisher bekannten Lehre in Mengen von mindestens 1:20 einsetzt.

Bei den erfindungsgemäßen Mengenverhältnissen von mindestens 1:20 erreicht man erstaunlicherweise eine wesentlich verbesserte Zieltier- und Anwender-Verträglichkeit und einen enormen wirkungssteigernden, synergistischen Effekt.

Die Erfindung betrifft daher Mittel, enthaltend

a) Flumethrin

b) MGK 264

in einem Gewichtsverhältnis der Komponenten a : b von mindestens 1:20 und höchstens 1:100,
sowie

c) gegebenenfalls weitere Wirkstoffe und

d) gegebenenfalls weitere Hilfs- und Trägerstoffe.

Die erfindungsgemäßen Mittel sind vorzugsweise fluid oder flüssig und eignen sich insbesondere hervorragend zur Herstellung von Spot-on- und Pour-on-Formulierungen für den Einsatz bei der Parasitenbekämpfung am Tier.

Wirkstoff (Komponente a) ist Flumethrin.

In den erfindungsgemäßen Mitteln liegt Flumethrin üblicherweise in Mengen von 0,01-20 Gew.%, bevorzugt 0,05-5,0 Gew.-%, besonders bevorzugt 0,075-0,75 Gew.-%, ganz besonders bevorzugt 0,10-0,50 Gew.-%, jeweils bezogen auf das Gewicht des fertigen Mittels vor. Im Falle von Sprayapplikationen sind die Konzentrationen üblicherweise geringer, und zwar liegen sie bevorzugt im Bereich 0,02 bis 0,1 Gew.%, besonders bevorzugt 0,03 bis 0,1 Gew.%, ganz besonders bevorzugt 0,03 bis 0,075 Gew.%.

Das Gewichtsverhältnis der Menge an Flumethrin zur Menge an MGK 264 liegt bei mindestens 1:20 ("mindestens" bedeutet hier, dass der Anteil an MGK 264 im Verhältnis zu Flumethrin auch höher sein kann), bevorzugt 1:30, besonders bevorzugt 1:40. Üblicherweise stellt man das Verhältnis nicht größer als 1:100, bevorzugt 1:80, besonders bevorzugt 1:60 ein.

Selbstverständlich können in den erfindungsgemäßen Mitteln weitere Wirkstoffe als Kombinationspartner eingesetzt werden.

Als Kombinationswirkstoffe seien bevorzugt genannt die im Bereich zur Bekämpfung von ektoparasitierenden Arthropoden. eingesetzten Insektizide wie Neonicotinoid-Insektizide, Spinosyne, N-Phenylpyrazole, Carbamate, Phosphor- und Phosphonsäureester, Wachstumshemmer sowie Mischungen dieser Wirkstoffe untereinander. Auch können weitere Synergisten zugesetzt werden. Als Synergisten im Sinne dieser Anmeldung werden Verbindungen verstanden, die selbst nicht die gewünschte Wirksamkeit aufweisen, als Mischpartner jedoch zu einer Steigerung der Wirksamkeit der aktiven Wirkstoffe führen.

Als Neonicotinoid-Insektizide seien genannt Verbindungen der Formeln (I), (II) und (III):

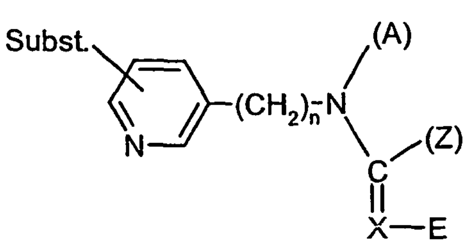

(I),

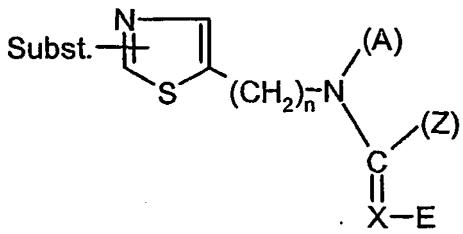 (II),

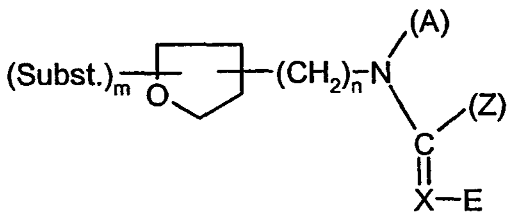 (III),

in welchen

n für 1 oder 2 steht,

m für 0, 1 oder 2 steht,

Subst. für einen der oben aufgeführten Substituenten, bevorzugt für Halogen, besonders bevorzugt für Chlor, steht,

A für eine monofunktionelle Gruppe aus der Reihe Wasserstoff, Acyl, Alkyl, Aryl steht oder für eine bifunktionelle Gruppe steht, die mit dem Rest Z verknüpft ist;

E für einen elektronenziehenden Rest steht;

X für die Reste -CH= oder =N- steht, wobei der Rest -CH= anstelle eines H-Atoms mit dem Rest Z verknüpft sein kann;

Z für eine monofunktionelle Gruppe aus der Reihe Alkyl, -O-R, -S-R, '

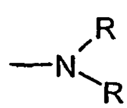 steht,

oder für eine bifunktionelle Gruppe steht, die mit dem Rest A oder dem Rest X verknüpft ist.

Bevorzugt sind Verbindungen der Formeln (I), (II) und (III), worin die Reste folgende Bedeutung haben:

A steht besonders bevorzugt für Wasserstoff sowie für gegebenenfalls substituierte Reste aus der Reihe $C_1$-$C_8$-Acyl, $C_1$-$C_{10}$-Alkyl, $C_6$-$C_{10}$-Aryl. A steht ferner für eine bifunktionelle Gruppe. Genannt sei gegebenenfalls substituiertes Alkylen mit 1-4, insbesondere 1-2 C-Atomen, wobei als Substituenten die weiter oben aufgezählten Substituenten genannt seien und wobei die Alkylengruppen durch 1 oder 2 gleiche oder verschiedenen Heteroatome aus der Reihe N, O, S unterbrochen sein können.

A und Z können gemeinsam mit den Atomen, an welche sie gebunden sind, einen gesättigten oder ungesättigten heterocyclischen Ring bilden. Der heterocyclische Ring kann weitere 1 oder 2 gleiche oder verschiedene Heteroatome und/oder Heterogruppen enthalten. Als Heteroatome stehen vorzugsweise Sauerstoff, Schwefel oder Stickstoff und als Heterogruppen N-Alkyl, wobei Alkyl der N-Alkyl-Gruppe vorzugsweise 1 bis 4, insbesondere 1 oder 2 Kohlenstoffatome enthält. Als Alkyl seien Methyl, Ethyl, n- und i-Propyl und n-, i- und t-Butyl genannt. Der heterocyclische Ring enthält 5 bis 7, vorzugsweise 5 oder 6 Ringglieder.
Als Beispiele für den heterocyclischen Ring seien Pyrrolidin, Piperidin, Piperazin, Hexamethylenimin, Hexahydro-1,3,5-triazin, Morpholin genannt, die gegebenenfalls bevorzugt durch Methyl substituiert sein können.

E steht für einen elektronentziehenden Rest, wobei insbesondere $NO_2$ CN, Halogenalkylcarbonyl, insbesondere mit 1-4 Kohlenstoffatomen und 1 bis 5 Halogenatomen, wie z.B. $COCF_3$, genannt seien.

X steht für -CH= oder -N=.

Z steht für gegebenenfalls substituierte Reste $C_1$-$C_{10}$-Alkyl, -OR, -SR, -NRR, wobei die Substituenten bevorzugt die bei R angegebene Bedeutung haben.

Z kann außer dem obengenannten Ring gemeinsam mit dem Atom, an welches es gebunden ist und dem Rest

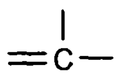

an der Stelle von X einen gesättigten oder ungesättigten heterocyclischen Ring bilden. Der heterocyclische Ring kann weitere 1 oder 2 gleiche - oder verschiedene Heteroatome und/oder Heterogruppen enthalten. Als Heteroatome stehen vorzugsweise Sauerstoff, Schwefel oder Stickstoff und als Heterogruppen N-Alkyl, wobei die Alkyl oder N-Alkyl-Gruppe vorzugsweise 1 bis 4, insbesondere 1 oder 2 Kohlenstoffatome enthält. Als Alkyl seien Methyl, Ethyl, n- und i-Propyl und n-, i- und t-Butyl genannt. Der heterocyclische Ring enthält 5 bis 7, vorzugsweise 5 oder 6 Ringglieder.
Als Beispiele für den heterocyclischen Ring seien Pyrrolidin, Piperidin, Piperazin, Hexamethylenimin, Morpholin und N-Methylpiperazin genannt.

R steht für Wasserstoff sowie für gegebenenfalls substituierte Reste aus der Reihe Acyl, Alkyl, Aryl, Aralkyl, Heteroaryl, Heteroarylalkyl.
AlsAcylresteseiengenanntFormyl,Alkylcarbonyl,Arylcarbonyl,Alkylsulfonyl,Arylsulfonyl,(Alkyl-)-(Aryl-)-phosphoryl, die ihrerseits substituiert sein können.
Als Alkyl seien genannt $C_{1-10}$-Alkyl, insbesondere $C_{1-4}$-Alkyl, im einzelnen Methyl, Ethyl, i-Propyl, sec.- oder t.-Butyl, die ihrerseits substituiert sein können.
Als Aryl seien genannt Phenyl, Naphthyl, insbesondere Phenyl.
Als Aralkyl seien genannt Phenylmethyl, Phenethyl.
Als Heteroaryl seien genannt Heteroaryl mit bis zu 10 Ringatomen und N, O, S insbesondere N als Heteroatomen. Im einzelnen seien genannt Thienyl, Furyl, Thiazolyl, Imidazolyl, Pyridyl, Benzthiazolyl.
Als Heteroarylalkyl seien genannt Heteroarylmethyl, Heteroarylethyl mit bis zu 6 Ringatomen und N, O, S, insbesondere N als Heteroatomen.
Als Substituenten seien beispielhaft und vorzugsweise aufgeführt:
Alkyl mit vorzugsweise 1 bis 4, insbesondere 1 oder 2 Kohlenstoffatomen, wie Methyl, Ethyl, n- und i-Propyl und n-, i- und t-Butyl; Alkoxy mit vorzugsweise 1 bis 4, insbesondere 1 oder 2 Kohlenstoffatomen, wie Methoxy, Ethoxy, n- und i-Propyloxy und n-, i-und t-Butyloxy; Alkylthio mit vorzugsweise 1 bis 4, insbesondere 1 oder 2 Kohlenstoffatomen, wie Methylthio, Ethylthio, n- und i-Propylthio und n-, i- und t-Butylthio; Halogenalkyl mit vorzugsweise 1 bis 4, insbesondere 1 oder 2 Kohlenstoffatomen und vorzugsweise 1 bis 5, insbesondere 1 bis 3 Halogenatomen, wobei die Halogenatome gleich oder verschieden sind und als Halogenatome, vorzugsweise Fluor, Chlor oder Brom, insbesondere Fluor stehen, wie Trifluormethyl; Hydroxy; Halogen, vorzugsweise Fluor, Chlor, Brom und Jod, insbesondere Fluor, Chlor und Brom; Cyano; Nitro; Amino; Monoalkyl- und Dialkylamino mit vorzugsweise 1 bis 4, insbesondere 1 oder 2 Kohlenstoffatomen je Alkylgruppe, wie Methylamino, Methyl-ethyl-amino, n- und i-Propylamino und Methyl-n-butylamino; Carboxyl; Carbalkoxy mit vorzugsweise 2 bis 4, insbesondere 2 oder 3 Kohlenstoffatomen, wie Carbomethoxy und Carboethoxy; Sulfo ($-SO_3H$); Alkylsulfonyl mit vorzugsweise 1 bis 4, insbesondere 1 oder 2 Kohlenstoffatomen, wie Methylsulfonyl und Ethylsulfonyl; Arylsulfonyl mit vorzugsweise 6 oder 10 Arylkohlenstoffatomen, wie Phenylsulfonyl sowie Heteroarylamino und Heteroarylalkylamino wie Chlorpyridylamino und

Chlorpyridylmethylamino.

Ganz besonders bevorzugt sind Verbindungen der Formeln (I), (II) und (III), worin

N für 1 steht,

m für 0 steht,

Subst. für Chlor steht,

A für Wasserstoff oder $C_{1-3}$-Alkyl steht,

Z für $C_{1-3}$-Alkyl, $NH_2$, -NH($C_{1-3}$-Alkyl) oder -N($C_{1-3}$-Alkyl)$_2$ steht,

oder

A und Z gemeinsam mit den Atomen, an welche sie gebunden sind, einen gesättigten 5- oder 6-gliedrigen heterocyclischen Ring bilden, der 1 oder 2 gleiche oder verschiedene Heteroatome oder Heterogruppen enthält, ausgewählt aus O, S, -NH-, -N($C_{1-3}$-Alkyl),

X für -CH= oder =N- steht,

E für $-NO_2$ oder CN steht.

Im einzelnen seien folgende Verbindungen genannt:

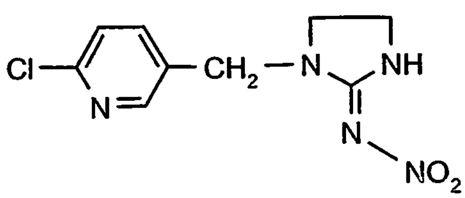

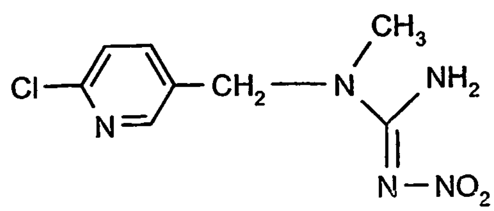

Imidacloprid

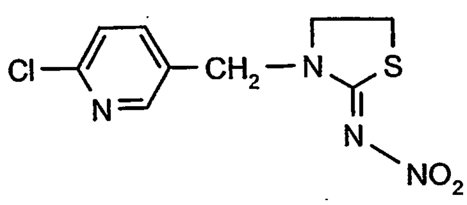

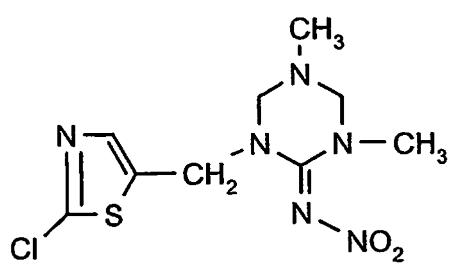

AKD 1022

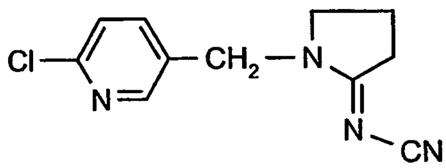

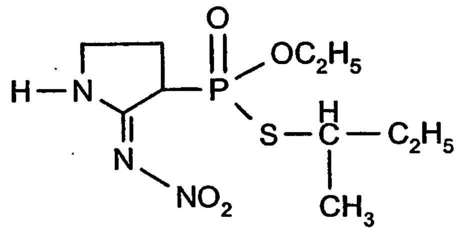

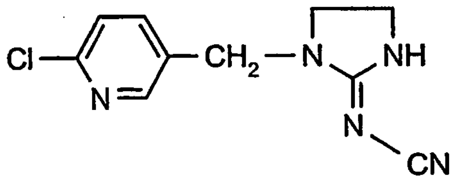

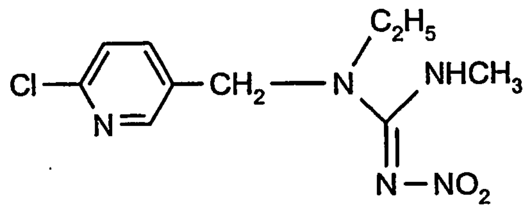

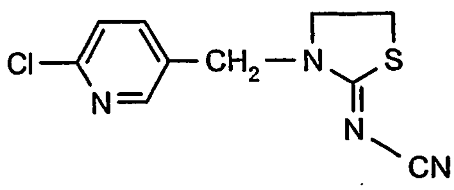

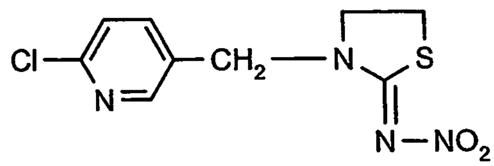

Thiacloprid

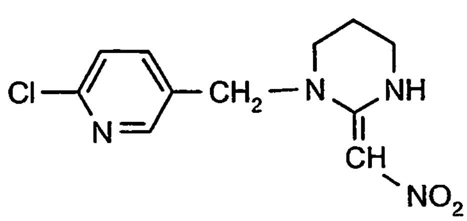

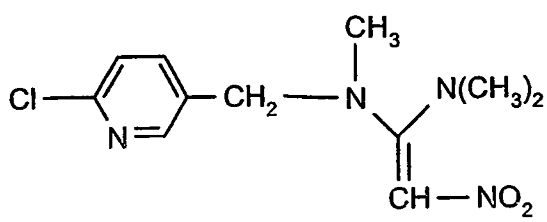

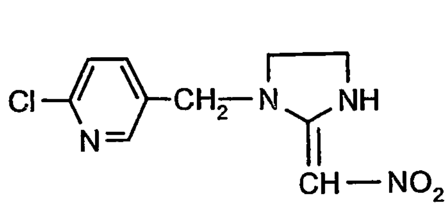

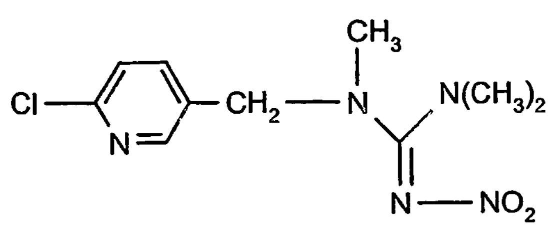

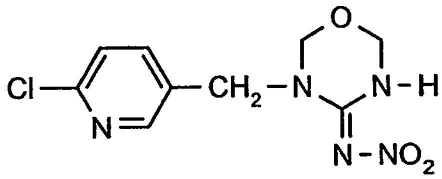

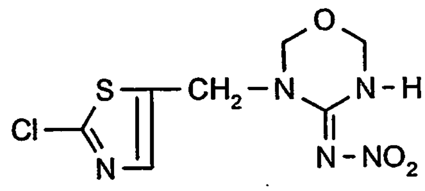

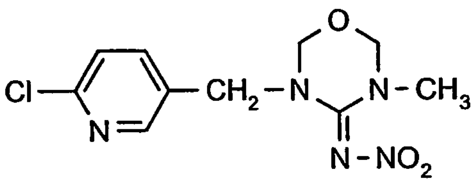

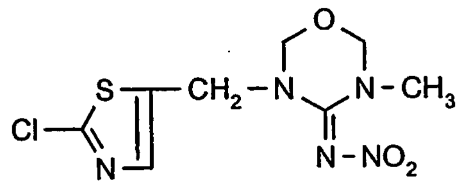

Thiamethoxam

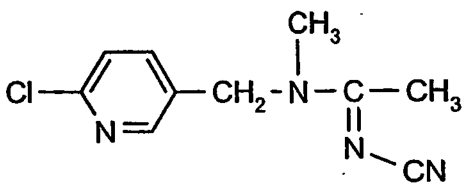

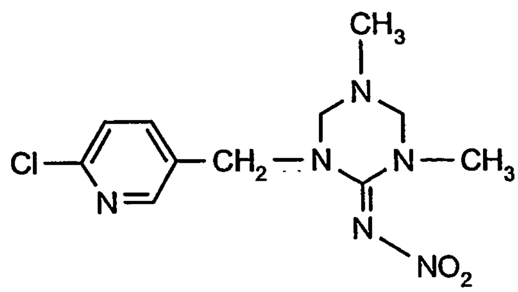

Acetamiprid

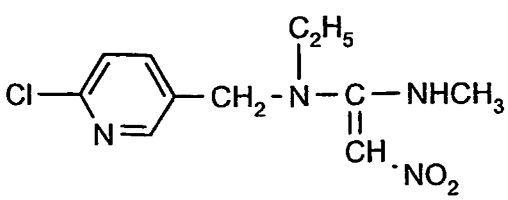

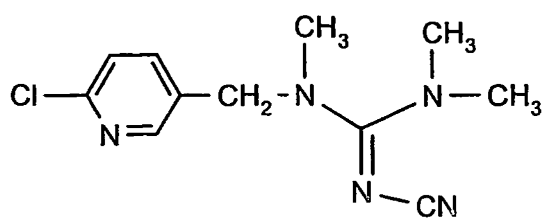

Nitenpyram

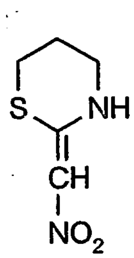

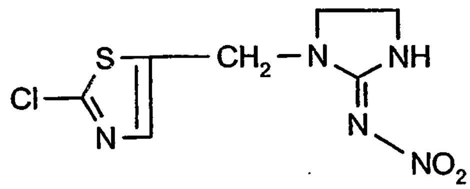

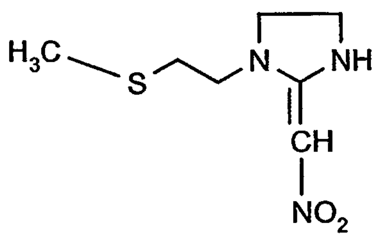

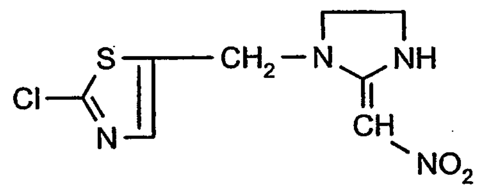

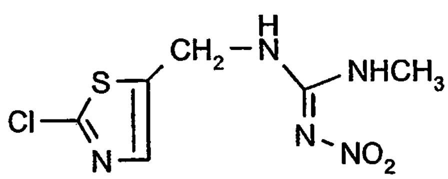

Chlothianidine

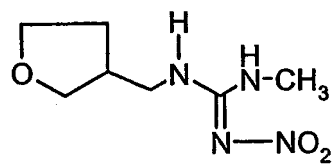

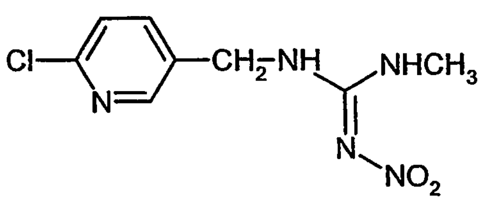

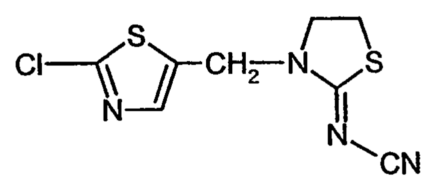

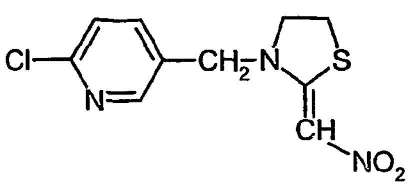

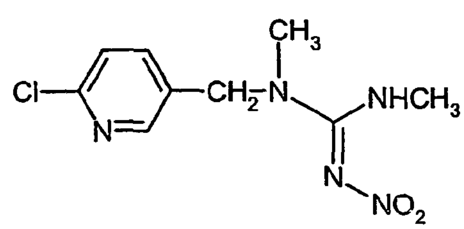

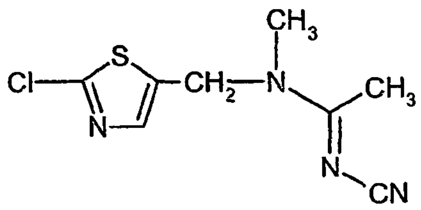

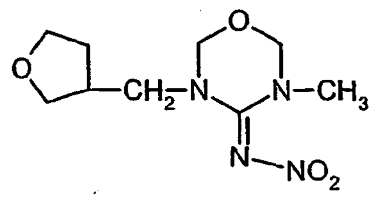

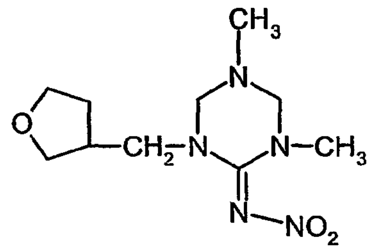

Besonders hervorgehoben seien die Verbindungen

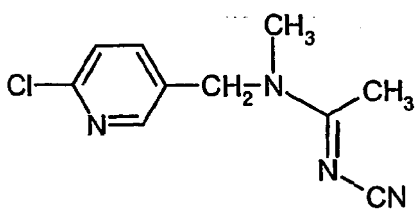

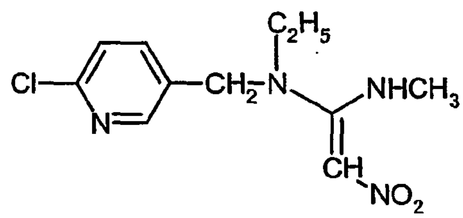

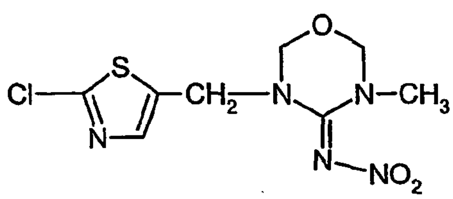

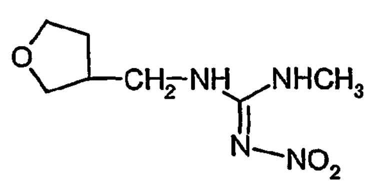

Weiterhin besonders hervorgehoben seien die Verbindungen

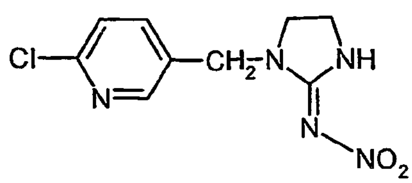

Imidacloprid

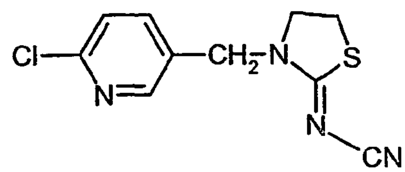

Thiacloprid

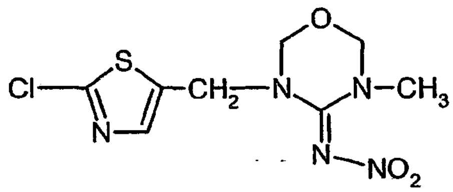

Thiamethoxam

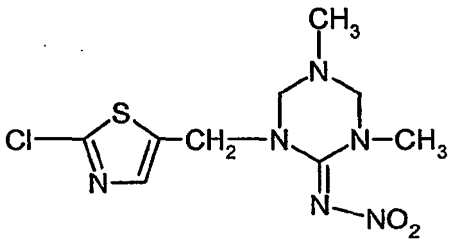

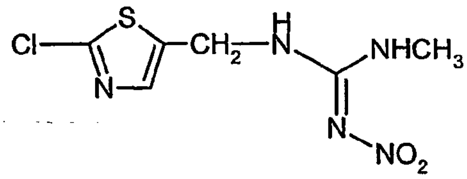

Als Spinosyne seien hier insbesondere genannt Spinosyn A und D

Spinosyn A

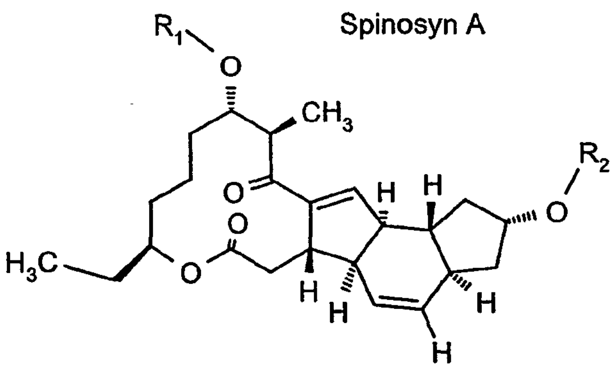

$R_1$ =

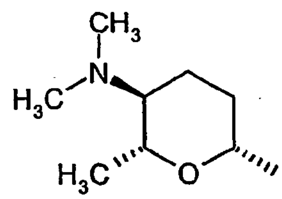

Spinosyn D

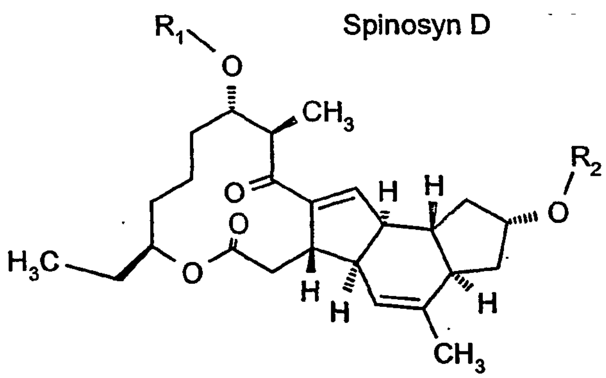

$R_2$ =

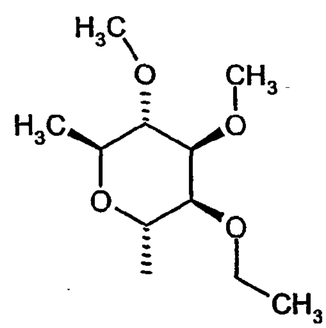

wie beschrieben in Boeck et al. in EP- 375 316 A1 and Deamicis et al. in WO 97/00265 A1.

Ebenfalls als Spinosyne werden hier verstanden synthetische und semi-synthetische Derivate der natürlichen Spinosyne bzw. Derivate die aus gentechnisch modifizierten Stämmen von z.B. Saccharopolyspora Spezies gewonnen werden, wie zum Beispiel beschrieben in WO 02/77004 und WO 02/77005.

Beispielhaft genannt seien Verbindungen der Formeln (IV) und (V) wobei $R_3$ ein Glykosid ($R_3 = R_1$) ist, $R_4$ ist H, OH oder Alkoxy; $R_5$ ist H, Methyl, $R_6$ und $R_7$ sind H oder zur Doppelbindung oder zu einer Epoxygruppe kombiniert, $R_8$ in Formel (IV) ist trans-1-Butenyl, 1,3-Butadienyl, Butyl, 3-Hydroxy-butenyl, Propyl, 1-Propenyl, 1,2-Epoxy-1-butyl, 3-Oxo-1-butenyl, $CH_3CH(OCH_3)CH{=}CH-$, $CH_3CH{=}CHCH(CH_2CO_2CH_3)-$, oder $CH_3CH{=}CHCH[CH_2CON(CH_3)]-$; $R_9$ ist H oder Glykosid ($R_9 = R_2$).

(IV)

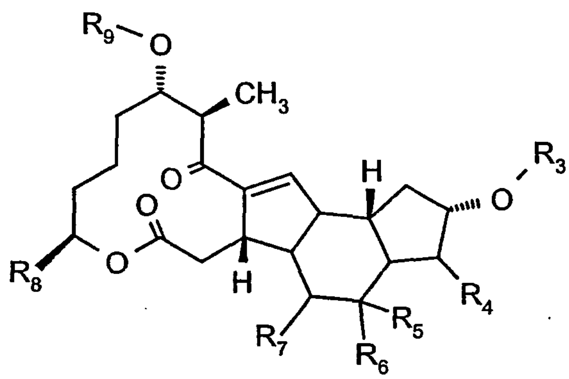

$R_1 =$

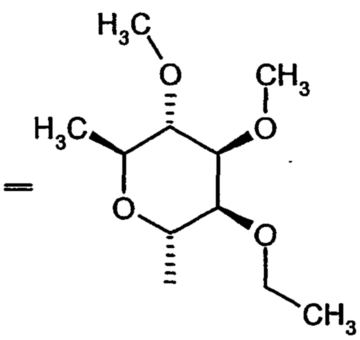

(V)

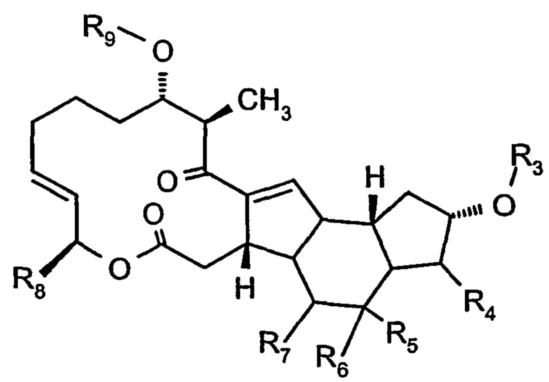

$R_2 =$

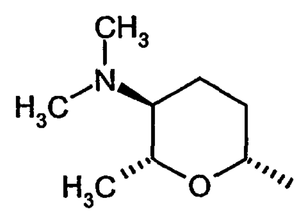

Als Phenylpyrazole seien zum Beispiel die folgenden Verbindungen genannt:

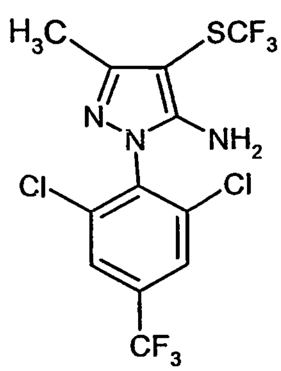

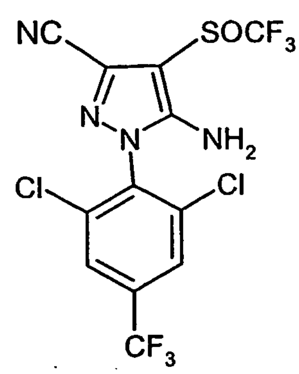

Fipronil

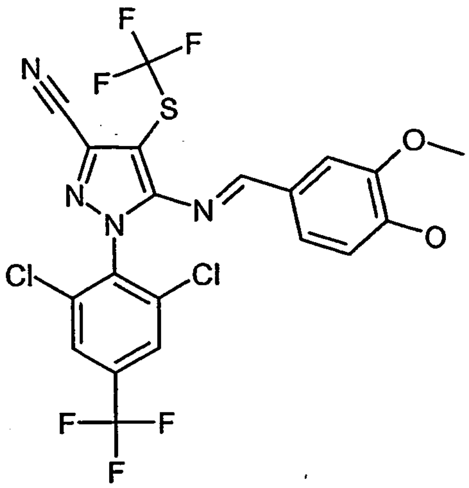

Vanilliprole,

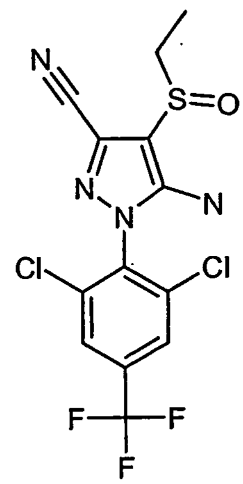

Ethiprole,

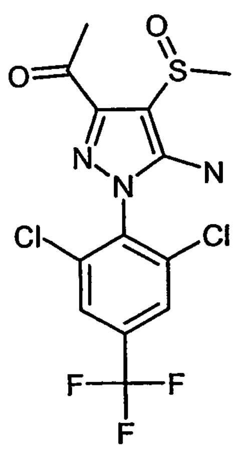

Acetoprole,

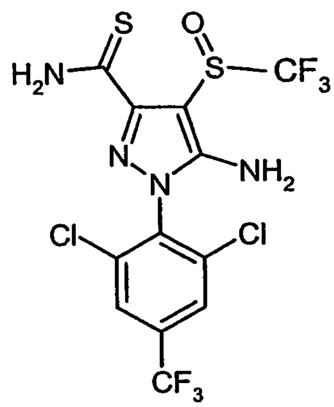

sowie Verbindungen aus WO 98/45274 zum Beispiel vom Typ

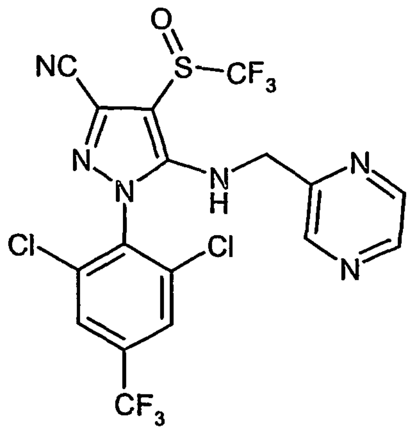

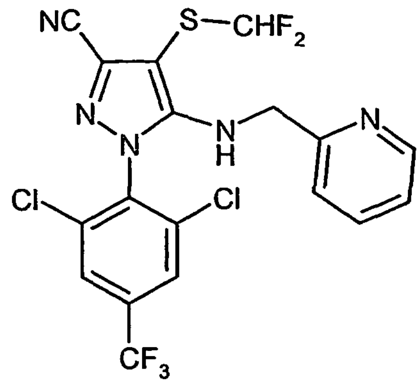

Als Carbamate seien genannt substituierte Phenyl- und Naphthylcarbamate, bevorzugte Beispiele sind:

- 2-Oxobutylphenyl-N-methylcarbamat,
- 4-Dimethylamino-3-methyl-phenyl-N-methylcarbamat,
- 2-isopropoxy-phenyl-N-methylcarbamat,
- 1-Naphthyl-N-methylcarbamat,
- m-Tolyl-N-methylcarbamat,
- 3,4-Xylyl-N-methylcarbamat,
- 3,5-Xylyl-N-methylcarbamat,
- 2-[1,3-Dioxolan-2-yl]-phenyl-N-methylcarbamat.

Als Phosphorsäureester seien bevorzugt genannt die Verbindungen mit den Common Names Phoxim, Fenitrothion, Dichlorvos, Trichlorfon und Malathion.

Juvenilhormone und juvenilhormonartige Verbindungen sind z.B. die folgenden:

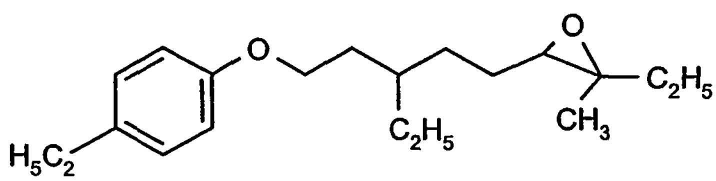

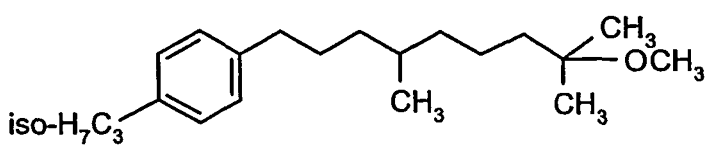

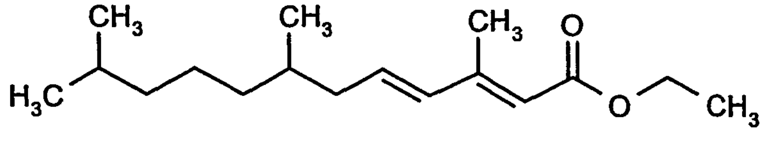

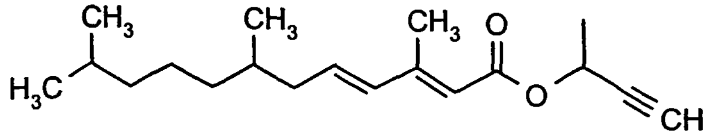

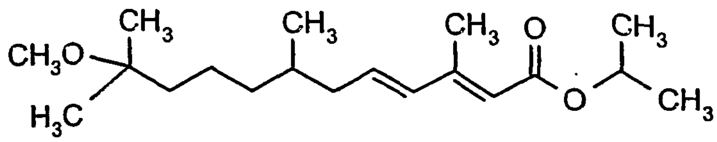

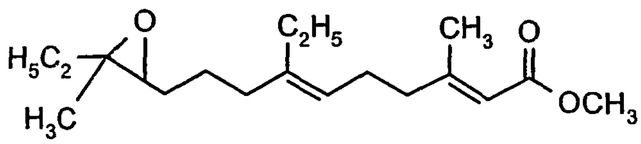

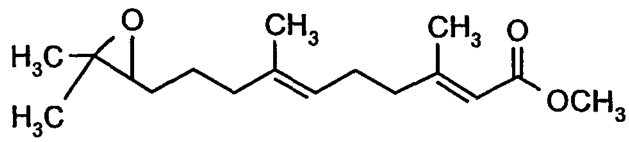

Substituierte Diarylether sind zum Beispiel die folgenden Verbindungen:

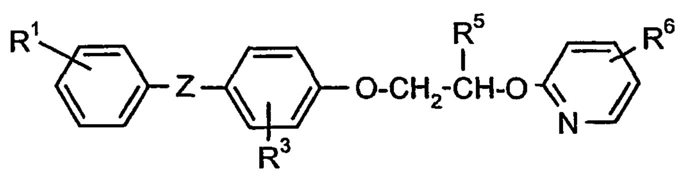

| $R^1$ | $R^3$ | $R^5$ | $R^6$ | Z |
|---|---|---|---|---|
| H | H | $CH_3$ | 2-Cl | O |
| 5-F | H | $CH_3$ | H | O |
| H | H | $CF_3$ | H | O |
| H | H | $C_2H_5$ | H | O |
| H | H | H | H | O |
| H | H | $CH_3$ | H | $CH_2$ |
| H | H | $CH_3$ | H | $C(CH_3)_2$ |

Benzoylharnstoffe sind z.B. die folgenden Verbindungen:

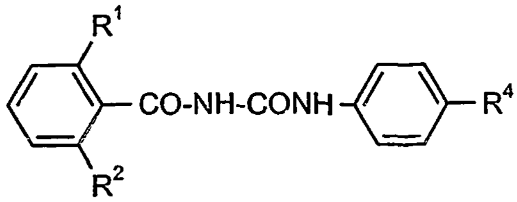

| $R^1$ | $R^2$ | $R^4$ |
|---|---|---|
| H | Cl | $CF_3$ |
| Cl | Cl | $CF_3$ |
| F | F | $CF_3$ |
| H | F | $CF_3$ |
| H | Cl | $SCF_3$ |
| F | F | SCF3 |
| H | F | $SCF_3$ |
| H | Cl | $OCF_3$ |
| F | F | $OCF_3$ |
| H | F | $OCF_3$ |
| F | F | 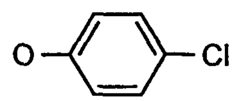 |
| F | F | 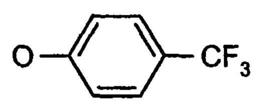 |
| F | F | 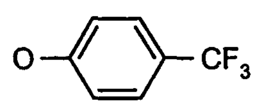 |

Triazine sind z.B. die folgenden Verbindungen:

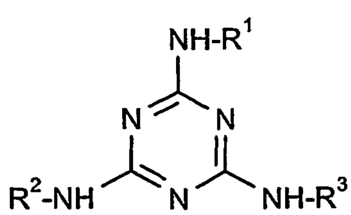

| $R^1$ | $R^2$ | $R^3$ |
|---|---|---|
| Cyclopropyl | H | H |
| Cyclopropyl | H | $CH_3$ |

(fortgesetzt)

| $R^1$ | $R^2$ | $R^3$ |
|---|---|---|
| Cyclopropyl | H | $C_2H_5$ |
| Cyclopropyl | H | $C_3H_7$-n |
| Cyclopropyl | H | $C_4H_9$-n |
| Cyclopropyl | H | $C_5H_{11}$-n |
| Cyclopropyl | H | $C_6H_{13}$-n |
| Cyclopropyl | H | $C_7H_{15}$-n |
| Cyclopropyl | H | $C_8H_{17}$-n |
| Cyclopropyl | H | $C_{12}$-$H_{25}$-n |
| Cyclopropyl | H | $CH_2$-$C_4H_9$-n |
| Cyclopropyl | H | $CH_2CH(CH_3)C_2H_5$ |
| Cyclopropyl | H | $CH_2CH{=}CH_2$ |
| Cyclopropyl | Cl | $C_2H_5$ |
| Cyclopropyl | Cl | $C_6H_{13}$-n |
| Cyclopropyl | Cl | $C_8H_{17}$-n |
| Cyclopropyl | Cl | $C_{12}H_{25}$-n |
| Cyclopropyl | H | Cyclopropyl |
| Cyclopropyl | H | $COCH_3$ |
| Cyclopropyl | H | $COCH_3$ HCl |
| Cyclopropyl | H | $COC_2H_5$ HCl |
| Cyclopropyl | H | $COC_2H_5$ |
| Cyclopropyl | H | $COC_3H_7$-n |
| Cyclopropyl | H | $COC_3H_7$-i |
| Cyclopropyl | H | $COC_4H_9$-t HCl |
| Cyclopropyl | H | $COC_4H_9$-n |
| Cyclopropyl | H | $COC_6H_{13}$-n |
| Cyclopropyl | H | $COC_{11}$-$H_{23}$-n |
| Cyclopropyl | $COCH_3$ | $COC_2H_5$ |
| Cyclopropyl | $COC_3H_7$-n | $COC_6H_{13}$-n |
| Cyclopropyl | $COCH_3$ | $COC_3H_7$-n |
| Cyclopropyl | $COC_2H_5$ | $COC_3H_7$-n |
| Cyclopropyl | H | COCyclopropyl |
| Cyclopropyl | COCyclopropyl | COCyclopropyl |
| Cyclopropyl | $COCH_3$ | $COCH_3$ |
| Isopropyl | H | H |
| Isopropyl | H | $COCH_3$ |
| Isopropyl | H | $COC_3H_7$-n |
| Cyclopropyl | H | $CONHCH_3$ |
| Cyclopropyl | H | $CONHC_3H_7$-i |

(fortgesetzt)

| $R^1$ | $R^2$ | $R^3$ |
|---|---|---|
| Cyclopropyl | $CONHCH_3$ | $CONHCH_3$ |
| Cyclopropyl | H | $SCNHCH_3$ |
| Cyclopropyl | H | $CONHCH_2CH=CH_2$ |
| Cyclopropyl | $CONHCH_2CH=CH_2$ | $CONHCH_2CH=CH_2$ |
| Cyclopropyl | $CSNHCH_3$ | $CSNHCH_3$ |

Insbesondere seien hier genannt Cyromazin und Dicylanil.

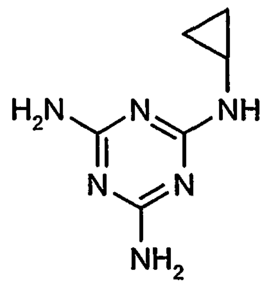
cyromazine

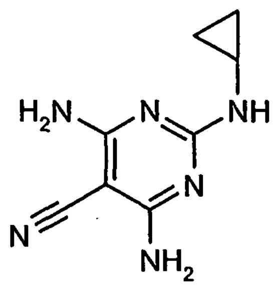
dicyclanil

Die Mengen der Kombinationswirkstoffe, die gegebenenfalls zusätzlich zu Flumethrin eingesetzt werden, können von 0,05 bis 25 % breit variiert werden, wobei die Mengen im Bereich 0,1 bis 15,0 % besonders und die Mengen im Bereich 0,5 bis 10,0 % ganz besonders zu bevorzugen sind. Prozentangaben sind hier als Gewichtsprozente bezogen auf die fertige Zubereitung zu verstehen.
Besonders bevorzugt sind Kombinationen des Flumethrin mit Neonicotinoiden, insbesondere Imidacloprid, Thiamethoxam, Clothianidin, Nitenpyram, Acetamiprid und Thiacloprid, oder mit Spinosynen, insbesondere Spinosad.
Selbstverständlich können den erfindungsgemässen Zubereitungen weitere Synergisten wie Piperonylbutoxid, Tributylphosphit und Sesamöl zugefügt werden. Diese Synergisten sind beispielsweise in EP-A 413 610 beschrieben.
Als Stabilisatoren und Antioxidantien seien genannt Sulfite oder Metabisulfite wie Kaliummetabisulfit; organische Säuren wie Citronensäure, Ascorbinsäure; Phenole, Butylhydroxytoluol, Butylhydroxyanisol, Tocopherol. Wobei die organischen Säuren Citronensäure und Äpfelsäure zu bevorzugen sind. Ganz besonders bevorzugte Stabilisatoren sind Citronensäure und Butylhydroxytoluol. Ihr Anteil kann im Bereich 0,05 bis 2,5 Gew.-% breit variiert werden. Wobei die Mengen im Bereich 0,075 bis 0,15 Gew.-% besonders bevorzugt werden. In Sprayformulierungen liegt die Untergrenze der üblichen Konzentrationen niedriger, in der Regel bei 0,01 Gew.-%, bevorzugt liegen in Sprayformulierungen die Konzentrationen bei 0,03 bis 0,1 Gew.%.
Zur Herstellung der erfindungsmäßigen Zubereitungen können aromatische Alkohole wie Benzylalkohol, cyclische Carbonate wie Propylen- und Ethylencarbonat, Pyrrolidone wie Pyrrolidon-2, N-Methylpyrrolidon, N-Oktyl-, N-Butyl-pyrrolidon, niedrigsiedende Alkohole wie Isopropanol, Ethanol, höhere Alkohole wie n-Octylalkohol, Lanolinalkohol und n-Butanol, cyklische und acyklische Ketone wie Aceton, Methylethylketon und Cyclohexanon, Glykole wie Ethylen- und Propylenglykol, aliphatische cyclische oder acyclische Ether wie Tetrahydrofurfurylalkohol, Diethylenglykolmonoethylether, Dipropylenglykolmonopropylether und Glycofurol, alyphatische oder aromatische Fettsäureester wie Isopropylmyristat, Isopropylpalmitat und Benzylbenzoat, Triglyceride auf Basis Ölsäure, Palmitinsäure, Linolsäure, Stearinsäure, Caprylsäure und Caprinsäure, Laktone wie Butyrolacton bzw. deren Mischungen untereinander eingesetzt werden. Besonders bevorzugt werden Carbonate, Alkohole und Pyrrolidone eingesetzt.
Der Lösungsmittelanteil der erfindungsgemäßen Mittel hängt selbstverständlich von der Art und Menge der weiteren Inhaltsstoffe ab und kann daher stark variieren. Üblicherweise beträgt der Lösungsmittelgehalt mindestens 10 Gew.-%, bevorzugt mindestens 50 Gew.-%, besonders bevorzugt mindestens 60 Gew.%.
Weiterhin können die erfindungsgemäßen Formulierungen polymere und/oder oligomere oberflächenaktive neutrale, kationische bzw. anionische Hilfsmitteln wie Polyvinylpyrrolidon, Polyvinylalkohol, Polyoxyethylen-, -oxypropylen-Sorbitansäureester, Polyoxyethylen-Stearate bzw. Umsetzungsprodukte der Phenoxyphenole und/oder Methoxysilanen mit Ethylenoxid und Propylenoxid, Alkali- und Erdalkalisalze der Carbon- und Sulfonsäuren, quarternäre Ammoniumsalze wie Benzylammoniumchlorid - gegebenenfalls auch in Kombination miteinander - in Mengen von 0,1 bis 5 Gew.%, vorzugsweise 0,2 bis 2,0 Gew.% enthalten, um das Fließverhalten, die Viskosität sowie Haar- und Fellaffinität zu verbessern.

Geruchsmaskierungsmittel sind z.B. Mischungen organischer Fettsäureester. Sie sind bevorzugt zu 0,1 bis 2 Gew.-% in den erfindungsgemäßen Formulierungen enthalten.

Falls die erfindungsgemäßen Mittel in Form eines Aerosolsprays eingesetzt werden, wird eine Vorlösung zusammen mit einem Treibmittel in übliche Spraydosen o.ä. abgefüllt. Übliche Treibmittel oder Treibgase sind z. B. gasförmige Kohlenwasserstoffe wie Propan, Butan (bevorzugt ist eine Propan-Butan-Mischung, insbesondere im Verhältnis 80:20), Fluorkohlenwasserstoffe, Fluorchlorkohlenwasserstoffe, $N_2O$, $CO_2$, Stickstoff.

Überraschenderweise zeichnen sich die erfindungsgemäßen Flüssigformulierungen durch eine hervorragende Lagerungsstabilität von mehreren Jahren in allen Klimazonen sowie durch ausgezeichnete Haut-, Anwender- und Umweltverträglichkeit aus. Sie eignen sich erstaunlicherweise auch hervorragend zum Abfüllen und Ausbieten in lagerungskritischen "Single dose Kunststofftuben", die üblicherweise aus Polypropylen bestehen, eine Wandstärke von 300-500 μm und ein Abfüllvolumen von 1,0 bis 4,0 ml aufweisen.

Solche mit den erfindungsgemäßen Mitteln befüllte Single-Dose-Kunststofftuben sind daher ebenfalls Gegenstand der vorliegenden Erfindung.

Die erfindungsgemäßen Flüssigformulierungen zeigen zudem einen nicht zu erwartenden synergistischen, d.h. wirkungssteigernden Effekt bei Verwendung von Flumethrin als Wirkstoff.

Die erfindungsgemäßen Mittel sind umweltverträglich und aufgrund der sehr geringen Toxizität anwenderfreundlich.

Die erfindungsgemäßen Mittel eignen sich bei günstiger Warmblütertoxizität zur Bekämpfung von parasitierenden Insekten, insbesondere Flöhen und Zecken, die bei Tieren, insbesondere bei Warmblütern, besonders bevorzugt bei Säugetieren vorkommen. Dies können Haus- und Nutztiere sowie Zoo-, Labor-, Versuchs- und Hobbytiere sein. Die erfindungsgemäßen Mittel sind dabei gegen alle oder einzelne Entwicklungsstadien der Schädlinge sowie gegen resistente und normal sensible Arten der Schädlinge wirksam.

Zu den Schädlingen gehören:

Aus der Ordnung der Anoplura z.B. Haematopinus spp., Linognathus spp., Solenopotes spp., Pediculus spp., Pthirus spp.;

aus der Ordnung der Mallophaga z.B. Trimenopon spp., Menopon spp., Eomenacanthus spp., Menacanthus spp., Trichodectes spp., Felicola spp., Damalinea spp., Bovicola spp;

aus der Ordnung der Diptera in der Unterordnung Brachycera z.B. Chrysops spp., Tabanus spp., Musca spp., Hydrotaea spp., Muscina spp., Haematobosca spp., Haematobia spp., Stomoxys spp., Fannia spp., Glossina spp., Lucilia spp., Calliphora spp., Auchmeromyia spp., Cordylobia spp., Cochliomyia spp., Chrysomyia spp., Sarcophaga spp., Wohlfartia spp., Gasterophilus spp., Oesteromyia spp., Oedemagena spp., Hypoderma spp., Oestrus spp., Rhinoestrus spp., Melophagus spp., Hippobosca spp..

aus der Ordnung der Diptera in der Unterordnung Nematocera z.B. Culex spp., Aedes spp., Anopheles spp., Culicoides spp., Phlebotomus spp., Simulium spp..

aus der Ordnung der Siphonaptera z.B. Ctenocephalides spp., Echidnophaga spp., Ceratophyllus spp., Pulex spp..

aus der Ordnung der Metastigmata z.B. Hyalomma spp., Rhipicephalus spp., Boophilus spp., Amblyomma spp., Haemaphysalis spp., Dermacentor spp., Ixodes spp., Argas spp., Ornithodorus spp., Otobius spp.;

aus der Ordnung der Mesostigmata z.B. Dermanyssus spp., Ornithonyssus spp., Pneumonyssus spp..

aus der Ordnung der Prostigmata z.B. Cheyletiella spp., Psorergates spp., Myobia spp., Demodex spp., Neotrombicula spp.;

aus der Ordnung der Astigmata z.B. Acarus spp., Myocoptes spp., Psoroptes spp., Chorioptes spp., Otodectes spp., Sarcoptes spp., Notoedres spp., Knemidocoptes spp., Neolmemidocoptes spp. Cytodites spp., Laminosioptes spp..

Besonders hervorgehoben sei die Wirkung gegen Siphonaptera, insbesondere gegen Flöhe und Zecken.

Zu den Nutz- und Zuchttieren gehören Säugetiere wie z.B. Rinder, Pferde, Schafe, Schweine, Ziegen, Kamele,

Wasserbüffel, Esel, Kaninchen, Damwild, Rentiere, Pelztiere wie z.B. Nerze, Chinchilla, Waschbär, Vögel wie z.B. Hühner, Gänse, Puten, Enten.

Zu Labor- und Versuchstieren gehören Mäuse, Ratten, Meerschweinchen, Goldhamster, Hunde und Katzen.

Zu den Hobbytieren gehören Hunde und Katzen.

Insbesondere hervorgehoben sei die Anwendung bei Katze und Hund.

Die Anwendung kann sowohl prophylaktisch als auch therapeutisch erfolgen.

Die erfindungsgemäß neuen Flüssigformulierungen sind grundsätzlich zur Spot on, Pour on, und Pump- sowie Aerosolspray-Applikationen geeignet. Die bevorzugten Applikationsformen sind Pour on, Pump Spray. Die Spot on Applikation ist ganz besonders bevorzugt.

Zur Herstellung der erfindungsgemäßen, flüssigen Formulierung werden die gewünschten Bestandteile in entsprechenden Mengen miteinander vermischt, z.B. durch den Einsatz konventioneller Rührkessel oder anderer geeigneter Geräte.

Falls die Inhaltsstoffe es erfordern, kann auch unter Schutzatmosphäre oder anderen Methoden des Sauerstoffausschlusses gearbeitet werden.

Die nachfolgenden Beispiele sollen die Erfindung erläutern:

## Beispiele

### Beispiel 1a

Eine homogene Spot on Formulierung (100 ml) bestehend aus

| | |
|---|---|
| 1,00 g | Flumethrin |
| 10,00g | Imidacloprid |
| 40,00 g | MGK 264 |
| 48,00 g | N-Methylpyrrolidon//THFA (Tetrahydrofurfurylalkohol) (70:30) |
| 0,10 g | Citronensäure |
| 0,10 g | BHT (Butylhydroxytoluol) |

### Beispiel 1b

Eine homogene Spot on Formulierung (100 ml) bestehend aus

| | |
|---|---|
| 0,50 g | Flumethrin |
| 10,00g | Imidacloprid |
| 40,00 g | MGK 264 |
| 48,50 g | N-Methylpyrrolidon//THFA (Tetrahydrofurfurylalkohol) (70:30) |
| 0,10 g | Citronensäure |
| 0,10 g | BHT (Butylhydroxytoluol) |

### Beispiel 2a

Eine homogene Spot on Formulierung (100 ml) bestehend aus

| | |
|---|---|
| 0,50 g | Flumethrin |
| 10,00g | Imidacloprid |
| 10,00 g | MGK 264 |
| 59,40g | N-Methylpyrrolidon//THFA (Tetrahydrofurfurylalkohol) (70:30) |
| 25,00g | Miglyol 812 |
| 0,10 g | Citronensäure |
| 0,10 g | BHT (Butylhydroxytoluol) |

### Beispiel 2b

Eine homogene Spot on Formulierung (100 ml) bestehend aus

| | |
|---|---|
| 0,35 g | Flumethrin |
| 10,00g | Imidacloprid |
| 10,00 g | MGK264 |
| 59,55 g | N-Methylpyrrolidon//THFA (Tetrahydrofurfurylalkohol) (70:30) |
| 25,00g | Miglyol 812 |
| 0,10 g | Citronensäure |
| 0,10 g | BHT (Butylhydroxytoluol) |

Beispiel 2c

Eine homogene Spot on Formulierung (100 ml) bestehend aus

| | |
|---|---|
| 0,20 g | Flumethrin |
| 10,00g | Imidacloprid |
| 10,00g | MGK 264 |
| 59,70g | N-Methylpyrrolidon//TEFA (Tetrahydrofurfurylalkohol) (70:30) |
| 25,00g | Miglyol812 |
| 0,10 g | Citronensäure |
| 0,10 g | BHT (Butylhydroxytoluol) |

Beispiel 3

Eine homogene Spot on Formulierung (100 ml) bestehend aus

| | |
|---|---|
| 0,50 g | Flumethrin |
| 10,00g | Imidacloprid |
| 20,00 g | MGK 264 |
| 50,00 g | N-Methylpyrrolidon//Propylencarbonat (70:30) |
| 13,75 g | Miglyol 812 |
| 0,1 g | Citronensäure |
| 0,1 g | BHT (Butylhydroxytoluol) |

Beispiel 4

Eine homogene Spot on Formulierung (100 ml) bestehend aus

| | |
|---|---|
| 0,50 g | Flumethrin |
| 10,00g | Imidacloprid |
| 10,00 g | MGK 264. |
| 72,85 g | Benzylalcohol |
| 14,48g | Propylencarbonat |
| 0,1 g | Citronensäure |
| 0,1 g | BHT (Butylhydroxytoluol) |

Beispiel 5

Eine homogene Spot on Formulierung (100 ml) bestehend aus

| | |
|---|---|
| 0,35 g | Flumethrin |
| 10,00 g | Imidacloprid |
| 10,00 g | MGK 264 |
| 72,70 g | Benzylalcohol |

(fortgesetzt)

| | |
|---|---|
| 14,33 g | Propylencarbonat |
| 0,1 g | Citronensäure |
| 0,1 g | BHT (Butylhydroxytoluol) |

Beispiel 6

Eine homogene Spot on Formulierung (100 ml) bestehend aus

| | |
|---|---|
| 0,35g | Flumethrin |
| 10,00 g | Imidacloprid |
| 15,00 g | MGK 264 |
| 65,46 g | Benzylalcohol |
| 12,94 g | Propylencarbonat |
| 0,1 g | Citronensäure |
| 0,1 g | BHT (Butylhydroxytoluol) |
| 5,00 g | Wasser |

Beispiel 7

Eine homogene Spot on Formulierung (100 ml) bestehend aus

| | |
|---|---|
| 0,35 g | Flumethrin |
| 10,00 g | Imidacloprid |
| 15,00 g | MGK 264 |
| 44,98 g | N-Methylpyrrolidon |
| 32,08 g | THFA |
| 0,1 g | Citronensäure |
| 0,1 g | BHT (Butylhydroxytoluol) |

Neben der besonders bevorzugten Spot-on Applikation ist bevorzugt auch eine Verteilung der genannten Wirkstoffkombinationen durch Sprühen mittels Pumpspray oder Aerosolspray möglich. Hierzu sind andere Formulierungen erforderlich, die in den nachfolgenden Herstellbeispielen beschrieben werden.

Beispiel 8

Eine Pumpsprayformulierung (250 ml) bestehend aus

| | |
|---|---|
| 2.50 g | Imidacloprid |
| 0.125 g | Flumethrin |
| 5.00 g | MGK 264 |
| 0.125 g | BHT |
| 0.125 g | Citronensäure |
| 20.80 g | Benzylalkohol |
| 4.125 g | Propylencarbonat |
| 25.00 g | Wasser |
| 154.63 g | Isopropanol |

Zur Applikation der Formulierung für die Wirksamkeitsstudien an Hunden und Katzen wurde ein konventioneller Pumpspray-Sprühkopf mit D(v0,5) von ca. 65μm eingesetzt.

Beispiel 9

Eine Pumpsprayformulierung (250 ml) bestehend aus

| | |
|---|---|
| 2.50 g | Imidacloprid |
| 0.125 g | Flumethrin |
| 5.00 g | MGK 264 |
| 0.125 g | BHT |
| 0.125 g | Citronensäure |
| 20.80 g | Benzylalkohol |
| 4.125 g | Propylencarbonat |
| 25.00 g | Wasser |
| 154.63g | Isopropanol |

Zur Applikation der Formulierung für die Wirksamkeitsstudien an Hunden und Katzen wurde ein konventioneller Pumpspray-Sprühkopf mit D(v0,5) von ca. 65μm eingesetzt.

Beispiel 10a

Eine Pumpsprayformulierung (250 ml) bestehend aus

| | |
|---|---|
| 2.50 g | Thiamethoxam |
| 0.125 g | Flumethrin |
| 5.00 g | MGK 264 |
| 0.125 g | BHT |
| 0.125 g | Citronensäure |
| 20.80 g | Benzylalkohol |
| 4.125 g | Propylencarbonat |
| 25.00 g | Wasser |
| 154.63 | g Isopropanol |

Zur Applikation der Formulierung für die Wirksamkeitsstudien an Hunden und Katzen wurde ein konventioneller Pumpspray-Sprühkopf mit D(v0,5) von ca. 65μm eingesetzt.

Beispiel 10b

Eine Pumpsprayformulierung (250 ml) bestehend aus

| | |
|---|---|
| 2.50 g | Thiacloprid |
| 0.125 g | Flumethrin |
| 5.00 g | MGK 264 |
| 0.125 g | BHT |
| 0.125 g | Citronensäure |
| 20.80 g | Benzylalkohol |
| 4.125 g | Propylencarbonat |
| 25.00 g | Wasser |
| 154.63 g | Isopropanol |

Zur Applikation der Formulierung für die Wirksamkeitsstudien an Hunden und Katzen wurde ein konventioneller Pumpspray-Sprühkopf mit D(v0,5) von ca. 65 μm eingesetzt.

Beispiel 11

Eine 250 ml Vorlösung zur Herstellung von üblichen Aerosol-Sprays bestehend aus

| | |
|---|---|
| 2.00 g | Thiamethoxam |
| 0.15 g | Flumethrin |
| 5.00 g | MGK 264 |
| 0.125 g | BHT |
| 0.025 g | Citronensäure |
| 36.475 g | Benzylalkohol |
| 7.225 g | Propylencarbonat |
| 25.00 g | Wasser |
| 141.25 g | Isopropanol |

Eine konventionelle Weissblech-Aerosoldose wurde mit 140 g der Vorlösung gemäß Beispiel 11 1 und mit 60 g einer Treibgasmischung Propan/Butan (Propan:Butan = 80:20) abgefühlt, mit einem üblichen Aerosol-Sprühkopf der Fa. Kosmos versehen und dann zur Durchführung von Wirksamkeitsstudien an Hunden und Katzen eingesetzt.

Beispiel 12

Eine 250 ml Vorlösung zur Herstellung von üblichen Aerosol-Sprays bestehend aus

| | |
|---|---|
| 2.00 g | Imidacloprid |
| 0.15 g | Flumethrin |
| 5.00 g | MGK 264 |
| 0.125 g | BHT |
| 0.025 g | Citronensäure |
| 36.475 g | Benzylalkohol |
| 7.225 g | Propylencarbonat |
| 25.00 g | Wasser |
| 141.25 g | Isopropanol |

Eine konventionelle Weissblech-Aerosoldose wurde mit 140 g der Vorlösung gemäß Beispiel 12 und mit 60 g einer Treibgasmischung Propan/Butan (Propan:Butan = 80:20) abgefüllt, mit einer herkömmlichen Aerosol-Sprühkopf der Fa. Kosmos versehen und dann zur Durchführung von Wirksamkeitsstudien an Hunden und Katzen eingesetzt.

Beispiel 13

Eine 250 ml Vorlösung zur Herstellung von üblichen Aerosol-Sprays bestehend aus

| | |
|---|---|
| 2.00 g | Thiacloprid |
| 0.15 g | Flumethrin |
| 5.00 g | MGK 264 |
| 0.125 g | BHT |
| 0.025 g | Citronensäure |
| 36.475 | g Benzylalkohol |
| 7.225 g | Propylencarbonat |
| 25.00 g | Wasser |
| 141.25 | g Isopropanol |

Eine konventionelle Weissblech-Aerosoldose wurde mit 140 g der Vorlösung gemäß Beispiel 13 und mit 60 g einer Treibgasmischung PropanlButan (Propan:Butan = 80:20) abgefüllt, mit einem üblichen Aerosol-Sprühkopf der Fa. Kosmos versehen und dann zur Durchführung von Wirksamkeitsstudien an Hunden und Katzen eingesetzt.

Der in den Beispielen 11- 13 eingesetzte Aerosol-Sprühkopf der Fa. Kosmos findet bei der Herstellung von handelsüblichen insektizidhaltigen Aerosol-Sprays (z.B. Bolfo Flohschutz Spray, Bolfo Plus Spray der Fa. Bayer HealthCare AG D-51368 Leverkusen) Anwendung.

Aus den weiteren Laborprüfungen zur Zecken-Wirksamkeit gemäß Beispielen 1, 2, 5 und 9 geht hervor, dass

die o.a. erfindungsgemäßen Formulierung eine sehr gute Wirkung gegen Zecken aufweisen, sich durch ihre Zieltier und Anwenderverträglichkeit auszeichnen und zum Bekämpfen von Flöhen Zecken an Kleintieren hervorragend geeignet sind.

A. Wirksamkeit gegen Flöhe am Hund

Ctenocephalides felis

An den Tagen -4 und -1 werden Hunde mit ca. 100 adulten, nüchternen Ctenocephalides felis pro Hund infestiert. Dabei werden die Flöhe auf den Nacken-des Tieres ausgebracht.
Am Tag 0 wird der Infestationserfolg am Hund überprüft, indem am wachen Tier nach Flöhen gesucht wird. Die Zahl der lebenden Flöhe wird protokolliert.
Nach der Zählung der Flöhe werden die Tiere behandelt. Die Hunde der Kontrollgruppe werden nicht behandelt. Die zu prüfenden Arzneimittel werden den Tieren dermal als Spot-on bei einer Applikationsmenge von 0,1 ml/kg Körpergewicht oder als Spray mit einer Applikationsmenge von 1-1.5 ml/kg Körpergewicht verabreicht. Die Applikation erfolgt einmalig am Tag 0. Es werden nur klinisch gesunde Tiere verwendet.
Am Tag 1 und 2 werden alle Hunde auf lebende Flöhe überprüft. Die Ergebnisse werden in den Rohdaten festgehalten.
Am Tag 7, 14, 21 und 28 werden alle Hunde mit ca. 100 adulten, nüchternen Ctenocephalides felis pro Hund reinfestiert. Jeweils einen Tag nach Reinfestation werden alle Hunde auf lebende Flöhe kontrolliert. Die Ergebnisse werden in den Rohdaten protokolliert.
Eine Formulierung wird als hochwirksam erachtet, wenn am Tag 1 und jeweils am zweiten Tag nach Reinfestation eine Wirksamkeit >95% festgestellt wird und diese Wirkung über mindestens 3-4 Wochen anhält.
Für die Berechnung der Wirksamkeit wird eine modifizierte Formel nach Abbott benutzt:

$$\text{Wirksamkeit \%} = \frac{\text{Ø Anzahl Flöhe KG} - \text{Ø Anzahl Flöhe BG}}{\text{Ø Anzahl Flöhe KG}} \times 100$$

KG: Kontrollgruppe

BG: Behandlungsgruppe

Die Arzneimittel gemäß den Formulierungsbeispielen 1 bis 5 in einer Dosierung von 0,1 ml/kg als Spot on appliziert, erwiesen sich gegen Ctenocephalides felis als hochwirksam.
Die Arzneimittel gemäß den Formulierungsbeispielen 8 bis 10 in einer Dosierung von 1 -1 .5mVkg als Spray appliziert, erwiesen sich gegen Ctenocephalides felis als hochwirksam.

B. Wirksamkeit gegen Zecken (Rhipicephalus sanguineus, Haemaphysalis leachi) am Hund

Jeweils an den Tagen -4 und -1 werden Hunde mit 2% Rompun® (Bayer AG, Wirkstoff: Xylazinhydrochlorid) (0,1ml/kg Körpergewicht) sediert. Nachdem alle Hunde sediert sind (nach ca. 10-15 Minuten) werden sie in Transportboxen überführt und 50 Rhipicephalus sanguineus oder Haemaphysalis leachi (25♀, 25♂) pro Hund auf den Nacken des Tieres ausgebracht. Die Tiere werden nach ca. 1 ½ Stunden wieder aus der Transportkiste in den Käfig gesetzt.
Am Tag 0 wird der Infestationserfolg am Hund überprüft, indem am wachen Tier nach Zecken gesucht wird. Intensiv wird dabei gesucht im Kopf und Ohrenbereich inkl. Ohrenfalte, im Bereich des Nackens, am Unterbauch, an der Unterbrust, an der seitlichen Flanke sowie zwischen den Zehen und an den Gliedmaßen. Die Zahl der angesogenen lebenden Zecken wird protokolliert. Tote Zecken werden entfernt.
Nach der Zählung der Zecken werden die Tiere behandelt. Die Hunde der Kontrollgruppe werden nicht behandelt. Die zu prüfenden Arzneimittel werden den Tieren dermal als Spot-on mit 0,1 ml/kg Körpergewicht oder als Spray mit 1-1.5 ml/kg Körpergewicht verabreicht. Die Applikation erfolgt einmalig am Tag 0. Es werden nur klinisch gesunde Tiere verwendet.
Am Tag 1 und Tag 2 werden alle Hunde auf lebende und tote angesogende Zecken überprüft. Die Ergebnisse werden in den Rohdaten festgehalten. Am Tag 2 werden alle lebenden und toten Zecken vom Hund entfernt.
Am Tag 7, 14, 21 und 28 werden alle Hunde mit jeweils 50 Rhipicephalus sanguineus oder Haemaphysalis leachi (25♀, 25♂) pro Hund reinfestiert. Jeweils zwei Tage nach Reinfestation werden alle Hunde auf lebende und tote

angesogene Zecken kontrolliert. Die Ergebnisse werden in den Rohdaten protokolliert. Am zweiten Tag nach Reinfestation werden alle lebenden und toten Zecken vom Hund entfernt.

Eine Formulierung wird als hochwirksam erachtet, wenn am Tag 2 und jeweils am zweiten Tag nach Reinfestation eine Wirksamkeit >90 % festgestellt wird und diese Wirkung über mindestens 3 Wochen anhält.

Für die Berechnung der Wirksamkeit wird eine modifizierte Formel nach Abbott benutzt:

$$\text{Wirksamkeit \%} = \frac{\text{Ø Anzahl Zecken KG} - \text{Ø Anzahl Zecken BG}}{\text{Ø Anzahl Zecken KG}} \text{ X } 100$$

KG: Kontrollgruppe

BG: Behandlungsgruppe

Die Arzneimittel in einer Dosierung gemäß den Formulierungsbeispielen 1 bis 5 von 0,1ml/kg als Spot on appliziert, erwiesen sich gegen Rhipicephalus sanguineus als hochwirksam.

Die Arzneimittel gemäß den Formulierungsbeispielen 8 bis 10 in einer Dosierung von 1-1.5 ml/kg als Spray appliziert, erwiesen sich gegen Rhipicephalus sanguineus und Haemaphysalis leachi als hochwirksam.

C. Wirksamkeit gegen Flöhe (Ctenocephalides felis) an der Katze

An Tag -1 werden Katzen mit ca. 100 adulten, nüchternen Ctenocephalides felis pro Katze infestiert. Dabei werden die Flöhe auf den Nacken des Tieres ausgebracht.

Am Tag 0 wird der Infestationserfolg an der Katze überprüft, indem am wachen Tier nach Flöhen gesucht wird. Die Zahl der lebenden Flöhe wird protokolliert.

Nach der Zählung der Flöhe werden die Tiere behandelt. Die Katzen der Kontrollgruppe werden nicht behandelt. Die zu prüfenden Arzneimittel gemäß der Beispiele 1 bis 4 werden den Tieren dermal als Spot-on bei einer Applikationsmenge von 0,1 ml/kg Körpergewicht verabreicht. Die Applikation erfolgt einmalig am Tag 0. Es werden nur klinisch gesunde Tiere verwendet.

An Tag 2 werden alle Katzen auf lebende Flöhe überprüft. Die Ergebnisse werden in den Rohdaten festgehalten.

Am Tag 6, 13, 20 und 27 werden alle Katzen mit ca. 100 adulten, nüchternen Ctenocephalides felis pro Katze reinfestiert. Jeweils zwei Tage nach Reinfestation werden alle Katzen auf lebende Flöhe kontrolliert. Die Ergebnisse werden in den Rohdaten protokolliert.

Eine Formulierung wird als hochwirksam erachtet, wenn am Tag 2 und jeweils am zweiten Tag nach Reinfestation eine Wirksamkeit >95% festgestellt wird und diese Wirkung über mindestens 3-4 Wochen anhält.

Für die Berechnung der Wirksamkeit wird eine modifizierte Formeln nach Abbott benutzt:

$$\text{Wirksamkeit \%} = \frac{\text{Ø Anzahl Flöhe KG} - \text{Ø Anzahl Flöhe BG}}{\text{Ø Anzahl Flöhe KG}} \text{ X } 100$$

KG: Kontrollgruppe

BG: Behandlungsgruppe

Die Arzneimittel gemäß den Formulierungsbeispielen 1 bis 5 in einer Dosierung von 0,1 ml/kg als Spot on appliziert, erwiesen sich gegen Ctenocephalides felis als hochwirksam.

D. Wirksamkeit gegen Zecken (Haemaphysalis leachi) an der Katze

Jeweils am Tag 2 werden Katzen mit einem milden Sedativum (Acepromazin maleat) sediert. Nachdem alle Katzen sediert sind (nach ca. 10-15 Minuten) werden 30 Haemaphysalis leachi (15♀, 15♂) pro Katze auf den Nacken des Tieres ausgebracht.

Am Tag -1 wird der Infestationserfolg an den Katzen überprüft, indem am wachen Tier nach Zecken gesucht

wird. Intensiv wird dabei gesucht im Kopf und Ohrenbereich, im Bereich des Nackens, am Unterbauch, an der Unterbrust, an der seitlichen Flanke sowie an den Gliedmaßen. Die Zahl der angesogenen lebenden Zecken wird protokolliert. Tote Zecken werden entfernt.

Nach der Zählung der Zecken werden die Tiere gruppiert. Die Behandlung erfolgt an Tag 0. Die Katzen der Kontrollgruppe werden nicht behandelt. Die zu prüfenden Arzneimittel werden den Tieren dermal als Spot-on mit 0,1 ml/kg Körpergewicht verabreicht. Die Applikation erfolgt einmalig am Tag 0. Es werden nur klinisch gesunde Tiere verwendet.

An Tag 2 werden alle Katzen auf lebende und tote angesogende Zecken überprüft. Die Ergebnisse werden in den Rohdaten festgehalten. Alle lebenden und toten Zecken werden von der Katze entfernt.

Am Tag 6, 13, 20 und 27 werden alle Katzen mit jeweils 30 Haemaphysalis leachi (15♀, 15♂) pro Katze reinfestiert. Jeweils zwei Tage nach Reinfestation werden alle Katzen auf lebende und tote angesogene Zecken kontrolliert. Die Ergebnisse werden in den Rohdaten protokolliert. Am zweiten Tag nach Reinfestation werden alle lebenden und toten Zecken von der Katze entfernt.

Eine Formulierung wird als hochwirksam erachtet, wenn am Tag 2 und jeweils am zweiten Tag nach Reinfestation eine Wirksamkeit >90 % festgestellt wird und diese Wirkung über mindestens 3 Wochen anhält.

Für die Berechnung der Wirksamkeit wird eine modifizierte Formel nach Abbott benutzt:

$$\text{Wirksamkeit \%} = \frac{\text{Ø Anzahl Zecken KG} - \text{Ø Anzahl Zecken BG}}{\text{Ø Anzahl Zecken KG}} \text{ X } 100$$

KG: Kontrollgruppe

BG: Behandlungsgruppe

Die Arzneimittel in einer Dosierung gemäß den Formulierungsbeispielen 1 bis 4 von 0,1ml/kg als Spot on appliziert, erwiesen sich gegen Haemaphysalis leachi als hochwirksam.

E. Floh- und Zeckenwirksamkeit über 4 bis 5 Wochen

Die Floh- und Zeckenwirksamkeit der erfindungsgemäßen Mittel wurde über vier bis fünf Wochen getestet. Die Versuchsdurchführung folgte der Beschreibung unter den Punkten A bis D.

<u>Tabelle 1</u> Floh- und Zeckenwirksamkeit des Mittels gemäß Beispiel 1a und 1b am Hund

| 1. Infestation: Tag -4 | 2. Infestation: Tag -1 | d 0 | Appl. Vol. | Woche 0 | | | | 3. Infestation: Tag 7 | Woche 1 | | 4. Infestation: Tag 14 | Woche 2 | | 5. Infestation: Tag 21 | Woche 3 | | 6. Infestation: Tag 28 | Woche 4 | | 7. Infestation: Tag 35 | Woche 5 | |
|---|---|---|---|---|---|---|---|---|---|---|---|---|---|---|---|---|---|---|---|---|---|---|
| | | Behandlung | ml/kg | d1 | | d2 | | | d9 | | | d16 | | | d23 | | | d30 | | | d37 | |
| | | | | Wirkung | | Wirkung | | | Wirkung | | | Wirkung | | | Wirkung | | | Wirkung | | | Wirkung | |
| | | | | CF % | RS % | CF % | RS % | | CF % | RS % | | CF % | RS % | | CF % | RS % | | CF % | RS % | | CF % | RS % |
| | | Beispiel 1a | 0,10 | 100 | 81 | 100 | 94 | | 100 | 100 | | 100 | 100 | | 100 | 98 | | 97 | 100 | | 95 | 95 |
| | | Beispiel 1b | 0,10 | 100 | 67 | 100 | 94 | | 100 | 96 | | 100 | 99 | | 100 | 100 | | 98 | 99 | | 92 | 98 |

Appl. Vol. = Aufgetragenes Volumen in ml / kg Körpergewicht

CF % = Wirksamkeit gegen Ctenocephalides felis Flöhe in %, berechnet über geometrische Mittelwertbestimmung gegenüber einer unbehandelten Kontrollgruppe

RS % = Wirksamkeit gegen Rhipicephalus sanguineus Zecken in %, berechnet über geometrische Mittelwertbestimmung gegenüber einer unbehandelten Kontrollgruppe

d = Tag

Tabelle 2 Floh- und Zeckenwirksamkeit des Mittels gemäß Beispiel 2a, 2b und 2c am Hund

| 1. Infestation: Tag -4 | 2. Infestation: Tag -1 | d 0 | Appl. Vol. | Woche 0 | | | | 3. Infestation: Tag 7 | Woche 1 | | | | 4. Infestation: Tag 14 | Woche 2 | | 5. Infestation: Tag 21 | Woche 3 | | 6. Infestation: Tag 28 | Woche 4 | | 7. Infestation: Tag 35 | Woche 5 | |
|---|---|---|---|---|---|---|---|---|---|---|---|---|---|---|---|---|---|---|---|---|---|---|---|---|
| | | | | d1 | | d2 | | | d8 | | d9 | | | d16 | | | d23 | | | d30 | | | d37 | |
| | | Behandlung | ml/kg | Wirkung | | Wirkung | | | Wirkung | | Wirkung | | | Wirkung | | | Wirkung | | | Wirkung | | | Wirkung | |
| | | | | CF % | RS % | CF % | RS % | | CF % | RS % | CF % | RS % | | CF % | RS % | | CF % | RS % | | CF % | RS % | | CF % | RS % |
| | | Beispiel 2a | 0.10 | 93 | 72 | 100 | 94 | | 100 | 100 | 100 | 100 | | 99 | 99 | | 100 | 100 | | 79 | 90 | | 83 | 89 |
| | | Beispiel 2b | 0.10 | 96 | 61 | 100 | 90 | | 100 | 97 | 99 | 98 | | 94 | 92 | | 75 | 85 | | 56 | 77 | | 13 | 33 |
| | | Beispiel 2c | 0.10 | 98 | 60 | 100 | 98 | | 100 | 100 | 100 | 99 | | 97 | 94 | | 94 | 95 | | 84 | 82 | | 58 | 54 |

Appl. Vol. = Aufgetragenes Volumen in ml / kg Körpergewicht

CF % = Wirksamkeit gegen Ctenocephalides felis Flöhe in %, berechnet über geometrische Mittelwertbestimmung gegenüber einer unbehandelten Kontrollgruppe

RS % = Wirksamkeit gegen Rhipicephalus sanguineus Zecken in %, berechnet über geometrische Mittelwertbestimmung gegenüber einer unbehandelten Kontrollgruppe

d = Tag

Tabelle 3 Floh- und Zeckenwirksamkeit des Mittels gemäß Beispiel 2b und 5 an der Katze

| 1. Infestation: Tag -2 | d 0 | Appl. Vol. | Woche 0 | | 2. Infestation: Tag 7 | Woche 1 | | 3. Infestation: Tag 14 | Woche 2 | | 4. Infestation: Tag 21 | Woche 3 | | 5. Infestation: Tag 28 | Woche 4 | |
|---|---|---|---|---|---|---|---|---|---|---|---|---|---|---|---|---|
| | Behandlung | ml/kg | d2 | | | d8 | | | d15 | | | d23 | | | d29 | |
| | | | Wirkung | | | Wirkung | | | Wirkung | | | Wirkung | | | Wirkung | |
| | | | CF % | HL % | | CF % | HL % | | CF % | HL % | | CF % | HL % | | CF % | HL % |
| | Beispiel 2b | 0.10 | 100 | 35 | | 100 | 67 | | 97 | 86 | | 91 | 80 | | 84 | 71 |
| | Beispiel 5 | 0.10 | 100 | 24 | | 100 | 93 | | 100 | 91 | | 99 | 91 | | 94 | 97 |

Appl. Vol. = Aufgetragenes Volumen in ml / kg Körpergewicht

CF % = Wirksamkeit gegen Ctenocephalides felis Flöhe in %, berechnet über geometrische Mittelwertbestimmung gegenüber einer unbehandelten Kontrollgruppe

HL % = Wirksamkeit gegen Haemaphysalis leachi Zecken in %, berechnet über geometrische Mittelwertbestimmung gegenüber einer unbehandelten Kontrollgruppe

d = Tag

Tabelle 4 Floh- und Zeckenwirksamkeit des Mittels gemäß Beispiel 9 am Hund

| 1. Infestation: Tag -4 | 2. Infestation: Tag -1 | d 0 | Appl. Vol. | Parasite | Woche 0 | 3. Infestation: Tag 7 | Woche 1 | 4. Infestation: Tag 14 | Woche 2 | 5. Infestation: Tag 21 | Woche 3 | 6. Infestation: Tag 28 | Woche 4 | 7. Infestation: Tag 35 | Woche 5 |
|---|---|---|---|---|---|---|---|---|---|---|---|---|---|---|---|
| | | | | | d2 | | d8 | | d14 | | d21 | | d28 | | d35 |
| | | Behandlung | ml/kg | | Wirkung | | Wirkung | | Wirkung | | Wirkung | | Wirkung | | Wirkung |
| | | Beispiel 9 | 1.30 | CF | 100 | | 100 | | 100 | | 100 | | 100 | | 100 |
| | | | | HL | 89 | | 99 | | 98 | | 94 | | 89 | | 74 |
| | | | | RS | 87 | | 99 | | 100 | | 98 | | 96 | | 91 |

Appl. Vol. = Aufgetragenes Volumen in ml / kg Körpergewicht

CF % = Wirksamkeit gegen Ctenocephalides felis Flöhe in %, berechnet über geometrische Mittelwertbestimmung gegenüber einer unbehandelten Kontrollgruppe

HL % = Wirksamkeit gegen Haemaphysalis leachi Zecken in %, berechnet über geometrische Mittelwertbestimmung gegenüber einer unbehandelten Kontrollgruppe

RS % = Wirksamkeit gegen Rhipicephalus sanguineus Zecken in %, berechnet über geometrische Mittelwertbestimmung gegenüber einer unbehandelten Kontrollgruppe

d = Tag

**Patentansprüche**

1. Mittel zur Bekämpfung von Parasiten an Tieren enthaltend

   a) Flumethrin
   b) MGK 264
   in einem Gewichtsverhältnis der Komponenten a : b von mindestens 1:20 und höchstens 1:100, sowie
   c) gegebenenfalls weitere Wirkstoffe und
   d) gegebenenfalls weitere Hilfs- und Trägerstoffe.

2. Mittel gemäß Anspruch 1, enthaltend ein Neonicotinoid-Insektizid als weiteren Wirkstoff.

3. Mittel gemäß Anspruch 1, enthaltend Imidacloprid.

4. Verwendung von Flumethrin in Kombination mit MGK 264 zur Herstellung von Mitteln gemäß Anspruch 1 zur Bekämpfung von Parasiten.

**Claims**

1. Compositions for controlling parasites on animals, comprising

   a) flumethrin
   b) MGK 264
   in a weight ratio of components a:b of at least 1:20 and at most 1:100, and also
   c) if appropriate further active compounds and
   d) if appropriate further auxiliaries and carriers.

2. Compositions according to Claim 1, comprising, as further active compound, a neonicotinoid insecticide.

3. Compositions according to Claim 1, comprising imidacloprid.

4. Use of flumethrin in combination with MGK 264 for preparing compositions according to Claim 1 for controlling parasites.

**Revendications**

1. Agents pour lutter contre des parasites des animaux contenant

   a) de la fluméthrine
   b) du MGK 264
   dans un rapport pondéral des composants a:b d’au moins 1:20 et de 1:100 au plus, ainsi que
   c) le cas échéant, d’autres substances actives et
   d) le cas échéant, d’autres excipients et vecteurs.

2. Agents selon la revendication 1, contenant un insecticide néo-nicotinoïde comme autre substance active.

3. Agents selon la revendication 1, contenant de l’imidaclopride.

4. Utilisation de la fluméthrine en association avec le MGK 264 pour préparer des agents selon la revendication 1 pour lutter contre des parasites.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 4874753 A
- EP 137627 A
- GB 2135886 A
- AU 627847
- EP 413610 A
- WO 9113545 A
- US 5466458 A
- WO 0135739 A
- US 0124306 A
- EP 981956 A
- US 6080796 A
- EP 981955 A
- US 6033731 A
- EP 375316 A1, Boeck
- WO 9700265 A1, Deamicis
- WO 0277004 A
- WO 0277005 A
- WO 9845274 A


### In der Beschreibung aufgeführte Nicht-Patentliteratur


- *JOURNAL OF ECONOMIC ENTOMOLOGY,* August 1994, vol. 87 (4), 879-84
- *JOURNAL OF ECONOMIC ENTOMOLOGY,* August 1987, vol. 80 (4), 728-32
- *India Chemosphere,* November 1997, vol. 35 (10), ISSN 0045-6535, 2365-2374
- *Japanese Journal of Sanitary Zoology,* 1995, vol. 46 (1), ISSN 0424-7086, 25-30
- *J ECON ENTOMOL, (1987),* 1987, vol. 80 (6), ISSN 0022-0493, 1117-1121
- *India Chemosphere,* 1998, vol. 36/15, 3055-3060
- **Wang I.-H. ; Moorman R. ; Burleson J.I-H. Wang.** *Journal of Liquid Chromatography and Related Technologies,* 1996, vol. 19/20, 3293-3304